# EUROPEAN PATENT SPECIFICATION

(11) **EP 4 114 843 B1**
(45) Date of publication and mention of the grant of the patent: **16.08.2023**
(21) Application number: 21743175.8
(22) Date of filing: 13.07.2021
(51) Int. Cl.: C07K 1/14, A61K 35/30, A61P 25/28, A23J 3/04, A23J 3/34, A23L 33/16, A23L 33/165, A23L 33/18, A61K 38/01

(54) **METHOD FOR PRODUCING A MAMMALIAN BRAIN PROTEIN HYDROLYSATE**
VERFAHREN ZUR HERSTELLUNG EINES SÄUGETIERHIRNPROTEINHYDROLYSATS
PROCÉDÉ DE PRODUCTION D'UN HYDROLYSAT DE PROTÉINES DE CERVEAU DE MAMMIFÈRE

(30) Priority: 13.07.2020 EP 20185414
(43) Date of publication of application: 11.01.2023
(73) Proprietor: EVER Neuro Pharma GmbH, 4866 Unterach am Attersee (AT)
(72) Inventor: HASELGRÜBLER, Thomas, 4866 Unterach am Attersee (AT); HUTTERER, Christian, 4866 Unterach am Attersee (AT); WINTER, Stefan, 4866 Unterach am Attersee (AT); SCHARTNER, Julia, 4866 Unterach am Attersee (AT); WINKLER, Juliane, 4866 Unterach am Attersee (AT)
(74) Representative: SONN Patentanwälte OG
(86) International application number: PCT/EP2021/069495
(87) International publication number: WO 2022/013236

(56) References cited:
- CN-A- 106 868 082
- RU-C1- 2 147 888
- WINDISCH M ET AL: "N-PEP-12 - a novel peptide compound that protects cortical neurons in culture against different age and disease associated lesions", JOURNAL OF NEURAL TRANSMISSION, vol. 112, no. 10, 1 October 2005 (2005-10-01), pages 1331-1343, XP019378003, ISSN: 1435-1463, DOI: 10.1007/S00702-005-0283-7 cited in the application

## Description

The present invention refers to methods for producing a mammalian brain protein hydrolysate, preferably for producing a porcine brain protein hydrolysate.

Pharmaceutical preparations of proteolytically generated neuropeptides of unique composition derived from purified brain proteins are disclosed in EP 0 452 299 A1, CN 103333941 A, WO 2005/074970 A2, WO 2007/128493 A1, RU 2 147 888 C1, CN 106 868 082 A, and Gromova et al. (Difficult Patient 8 (2010), 25-31). Such preparations usually comprise 15 to 30% low molecular weight peptides (< 10kDa) and free amino acids such as alanine, aspartic acid, arginine, glutamic acid, glycine, histidine, isoleucine, leucine, lysine, methionine, phenylalanine, proline, serine, threonine, tryptophan, cysteine and valine. These solutions are preferably sterile and ready for injection or infusion (i.e. it fulfils all standards and requirements for pharmaceutical preparations to be administered to human patients). Such brain protein hydrolysates are e.g. a mixture of short-chain peptides purified from pig brains, which may including peptides mimicking (and not limited to) brain-derived neurotrophic factor (BDNF), glial cell line-derived neurotrophic factor (GDNF), nerve growth factor (NGF), and ciliary neurotrophic factor (CNTF) (Chen et al., Neurobiol. Aging 28 (2007), 1148-1162; Hartbauer et al., J Neural Transm. 108 (2001), 459-473 and 581-592) .

N-PEP-12 is an orally available drug derived from a peptide preparation produced enzymatically from purified nerve cell proteins, i.e. from a brain protein hydrolysate preparation but of lower potency and area of usage (Windisch et al., J. Neur. Transmission 112 (2005), 1331-1343). N-PEP-12 consists of 70% peptides based on total nitrogen content and 30% free amino acids, and thus represents a peptide-enriched derivative of a brain protein hydrolysate preparation.

Such brain protein hydrolysates may be provided as a peptide mixture with neurotrophic-like properties that ameliorates behavioural and cognitive deficits in preclinical models and patients with dementia; and improves motor function, cognitive performance and activities of daily living in stroke as well as in TBI. The mode of action of such brain protein hydrolysates are pleiotrophic, ranging from neuroprotective properties such as reduced excitotoxicity and free radical formation to enhanced recovery via increased neuroplasticity and neurogenesis.

Such brain protein hydrolysates are used for treatment of stroke and vascular dementia. For example, a beneficial effect on cognitive function in people with vascular dementia, possibly through decreased beta-amyloid deposition was reported. Brain protein hydrolysates are approved to treat stroke in over 50 countries world-wide. Furthermore, brain protein hydrolysates are used for the treatment of CADASIL (for cerebral autosomal dominant arteriopathy with subcortical infarcts and leukoencephalopathy; or: CADASIL syndrome), especially for reducing mortality in CADASIL patients (WO 2019/042983 A1).

Although such brain protein hydrolysates are therefore already a successfully marketed product, it is a desire to investigate whether an extension of this product to the field of nutritional supplements is possible and, if yes, how such improved nutritional supplement products (such as NPEP-12) or can be provided in an effective manner (and in an orally available active form). It is therefore an objective to provide extended production processes for mammalian brain protein hydrolysates which are, optionally, also suitable for other products which are similar to NPEP-12, i.e. protein hydrolysate products. It is a further objective to provide novel mammalian brain protein hydrolysate preparations which show extension in relevant properties and functions compared to known mammalian brain protein hydrolysate products, especially with respect to effects in the neural system, such as improved effects on the integrity of cerebral endothelia cells (or the blood brain barrier (BBB)). Further objects of the present invention are the establishment of an orally available mammalian brain protein hydrolysate product which may even be used as a nutritional supplement. It is a further object to use such novel products for the prevention, amelioration and, if applicable, treatment of age associated memory impairment (AAMI).

Therefore, the present invention provides a method for the production of a mammalian brain protein hydrolysate, preferably for producing an improved nutritional supplement product derived from such mammalian brain protein hydrolysate product, comprising the following steps:
- providing a mammalian brain protein hydrolysate,
- adding Zn ions to the mammalian brain protein hydrolysate to form a complex comprising the Zn ions and the protein hydrolysate,
- obtaining the complex comprising the Zn ions and the mammalian brain protein hydrolysate in a mammalian brain protein hydrolysate composition.

The present invention provides a method for the production of mammalian brain protein hydrolysate compositions which has been specifically developed for extending the availability and areas of use of mammalian brain protein hydrolysate compositions.

For achieving high zinc concentrations and readily transfer of Zinc ions to peptides and amino acids at neutral pH it is beneficial to use zinc sources such as Zn gluconate, Zn sulfate or Zn acetate, preferably Zn gluconate. Zinc gluconate is the zinc salt of gluconic acid. It is an ionic compound consisting of two anions of gluconate for each zinc (II) cation (Zn²⁺). In Zn gluconate, the Zn ion forms a weak complex with gluconate. In the method according to the present invention, the Zn ions are added as Zn gluconate, preferably in amount of 0.1 to 500 mg/ml, preferably from 0.5 to 100 mg/l, especially from 1 to 70 mg/ml.

The Zn complexed composition according to the present invention can be orally administered and exhibits novel and extended effectiveness e.g. in comparison with prior art products, such as N-PEP (Windisch et al., 2005; "NPEP-non Z" in the example section), or in comparison with non-complexed, Zn containing hydrolysates ("NPEP-low pH" in the example section), as demonstrated e.g. in endothelial permeability tests or in reducing pro-inflammatory proteins (see e.g. example section, below).

In a preferred embodiment of the present method, the mammalian brain protein hydrolysate is a hydrolysate of porcine or bovine brain tissue, preferably an intermediate of the manufacturing process, especially a peptide-enriched intermediate product. According to a preferred embodiment, the intermediate is a purified hydrolysate that lacks high molecular weight components such proteins or nucleic acids and fatty acids (i.e. not detectable, e.g. less than 10ng/dose nucleic acids). Preferred mammalian brain protein hydrolysates are disclosed in EP 0 452 299 A1, WO 2005/074970 A2, and WO 2007/128493 A1. Accordingly, the mammalian brain protein hydrolysate preferably contains at least one peptide selected from the group NMVPFPR, ASAFQGIGSTHWVYDGVGNS, and DLHW.

According to a preferred embodiment, the mammalian brain protein hydrolysate is an enzymatically derived mammalian brain hydrolysate. Although also other hydrolysis methods are useable, such as basic, acidic or mechanical hydrolysis, enzymatic hydrolysis is preferred, mainly due to its standardisation eligibility, its convenience in handling, and its reproducibility. Preferred enzymes to obtain enzymatic hydrolysis are enzymes which are obtainable in large and high quality and reproducibility on the industrial market, preferably enzymes, such as trypsin, pepsin, chymotrypsin, papain, carboxypeptidase, or mixtures thereof (e.g. pancreatin).

Preferably, the Zn ions are added to the mammalian brain protein hydrolysate at a pH of 2.5 to 7.5, preferably of 3.0 to 6.5.

In a preferred embodiment of the present method, the pH of the mammalian brain protein hydrolysate composition changes upon formation of the complex comprising the Zn ions and the mammalian brain protein hydrolysate.

Preferably, the final pH of the mammalian brain protein hydrolysate composition is adjusted (and kept for the final product) to 3.0 to 9.0, especially from 5.0 to 9.0. According to a preferred embodiment, the final product is provided with a roughly neutral to slightly basic pH ("roughly neutral"; for physiological reasons); the pH is therefore preferably adjusted to 6.0 to 8.0, especially 7.0 to 7.5.

According to an alternatively preferred embodiment, a more acidic product is provided with a pH of 3.0 to 6.0, preferably 3.5 to 5.0, especially 4.0 to 5.0. A specifically preferred product has a pH of about 4.7 (e.g. of 4.5 to 4.9). This preferred "acidic" embodiment has procedural advantages, specifically for mass production and storage reasons.

The relative amounts of the Zn ions and the mammalian brain protein hydrolysate may be optimised for each hydrolysate, preferably (and specifically suitable for forming the Zn complexed brain protein hydrolysate according to the present invention), the mammalian brain protein hydrolysate provided contains from 0.5 to 100 nitrogen (N₂) mg/ml, preferably 1 to 50 mg/ml, more preferred 5 to 30 mg/ml, especially 5 to 20 mg/ml.

According to a preferred embodiment, the Zn ions are added to the mammalian brain protein hydrolysate in a relative amount of 0.05 to 50 mg Zn per mg mammalian brain protein hydrolysate (as mg N₂), preferably of 0.1 mg to 10 mg Zn per mg mammalian brain protein hydrolysate (as mg N₂), especially 0.2 mg to 4 mg Zn per mg mammalian brain protein hydrolysate (as mg N₂). Nitrogen content may be analysed by elementary analysis or by the Kjeldahl method (e.g. European Pharmacopoeia (Ph.Eur.), 2.5.9. Determination of nitrogen). Amino acid content is analysed by HPLC analysis; peptide content is calculated from the free amino acid content and the nitrogen content.

According to a preferred embodiment of the present method, the Zn concentration in the Zn complexed mammalian brain protein hydrolysate is adjusted to a concentration of 0.05 to 50 mg/ml, preferably from 0.1 to 25 mg/ml, especially from 0.2 to 20 mg/ml.

According to a preferred embodiment of the present method, the Zn complexed mammalian brain hydrolysate is finished to a final preparation, preferably to a nutritional supplement or to a pharmaceutically acceptable preparation, especially preferably by filling the Zn complexed protein hydrolysate into a nutritionally acceptable dosage form or into a pharmaceutically acceptable container.

The present invention represents a method for the production of a mammalian brain protein hydrolysate in which peptides and amino acids are complexed with zinc ions.

Zinc ions are preferentially added as zinc gluconate because of its good water solubility up to pH 7.5 due to weak complex formation. Other zinc salts such as zinc sulfate and zinc acetate may also be used.

In the method according to the present invention, zinc ions are preferably added in an amount of 1 to 50 mg/ml, preferably 2 to 20 mg/ml or as a solid powder to the mammalian brain protein hydrolysate having a preferred total nitrogen content of 1 - 50 mg/ml, especially 5 - 25 mg/ml.

Preferably, the Zn ions are added to the mammalian brain protein hydrolysate at a pH of 2.5 to 7.5. The final pH is preferably adjusted (in the "roughly neutral" preferred embodiment) to 6.0 - 8.0, especially 7.0 - 7.5 to achieve a high degree of complex formation. In the "acidic" preferred embodiment, the final pH is adjusted to a pH of 3.0 to 6.0, preferably of 3.5 to 5.0, especially of 4.0 to 5.0 (specifically to a pH of about 4.7 (e.g. of 4.5 to 4.9)), again, also i.a. to achieve a high degree of complex formation.

The preferred uses of the products made according to the present invention are the use as a nutritional supplement and a pharmaceutical use. Accordingly, the Zn complexed protein hydrolysate according to the present invention is preferably finished to a final preparation (allowing marketing of the product), preferably to a nutritional supplement or to a pharmaceutically acceptable preparation, preferably by filling the Zn complexed protein hydrolysate into a nutritionally acceptable dosage form or into a pharmaceutically acceptable container.

Preferably, the Zn complexed mammalian brain protein hydrolysate is dried, preferably spray-dried, fluid bed dried or lyophilised.

According to a preferred embodiment, the method according to the present invention comprises admixing further components with additional substances before, after or during the complex formation. Preferred additional substances are formulation additives, stabilizers, nutritional additives, pharmaceutically acceptable excipients, or mixtures thereof, especially carbohydrates, such as lactose, mannitol, maltodextrin, sucrose, dextran, trehalose, cellulose, hydrolysed starch, and mixtures thereof (which may be used for all purposes, especially as spray drying, fluid bed drying formulation additives and stabilizers (Lee et al., Food Res. 2 (2018), 486 -499)).

Preferably, the Zn complexed mammalian brain protein hydrolysate is dried, preferably spray-dried, fluid bed dried or lyophilised, and may then be brought into a final container (or may be lyophilized already in the final container).

According to another aspect, the present invention refers to composition comprising the new Zn complexed mammalian brain protein hydrolysate provided by the present invention, preferably obtainable according to the method according to the present invention.

For the present invention, preferred hydrolysates are enriched in peptides and contain - based on total nitrogen content - 50 to 90 % peptides and 50 to 10 % free amino acids, preferably 60 to 80 % and 40 to 20 % free amino acids.

Based on total dry weight, preferred compositions according to the present invention comprise 5 to 90 % peptides, 1 to 90 % free amino acids and 1 to 90 % additional substances, such as formulation additives stabilizers, nutritional additives, pharmaceutically acceptable excipients, or mixtures thereof.

Specifically preferred nutritional supplement compositions according to the present invention contain 10 to 50 %, preferably 10 to 30%, peptides, 1 to 30 %, preferably 2 to 20%, free amino acids and 20 to 89 %, additional substances, especially carbohydrates, such as lactose, mannitol, maltodextrin, sucrose, dextran, trehalose, cellulose, hydrolysed starch, and mixtures thereof.

The present invention is specifically drawn to the provision of improved nutritional supplement products based on the present Zn complexed mammalian brain protein hydrolysate and the use of the present Zn complexed mammalian brain protein hydrolysates as a nutritional supplement. The term "nutritional supplement" or "food supplement" (to be used interchangeably within the context of the present invention) is to be interpreted for the present invention according to the EU Directive 2002/46/EC of 10 June 2002 as "foodstuffs the purpose of which is to supplement the normal diet and which are concentrated sources of nutrients or other substances with a nutritional or physiological effect, alone or in combination, marketed in dose form, namely forms such as capsules, pastilles, tablets, pills and other similar forms, sachets of powder, ampoules of liquids, drop dispensing bottles, and other similar forms of liquids and powders designed to be taken in measured small unit quantities".

Accordingly, preferred dose forms of the nutritional supplements according to the present invention are selected from the group of capsules, pastilles, tablets, pills and other similar forms, sachets of powder, ampoules of liquids, drop dispensing bottles, and other similar forms of liquids and powders designed to be taken in measured small unit quantities.

The nutritional supplements comprising the present Zn complexed mammalian brain protein hydrolysates are therefore i.a. intended to supplement the diet and provide nutrients that may be missing or may not be consumed in sufficient quantities in a person's diet.

According to a preferred aspect, the present invention relates to a nutritional supplement comprising a Zn complexed mammalian brain protein hydrolysate, preferably obtainable according to the method according to the present invention.

The nutritional supplement according to the present invention is preferably provided in dose form. Such dose forms are those typically used for nutritional supplements (food supplements), i.e. as capsules, pastilles, tablets, pills and other similar forms, sachets of powder, ampoules of liquids, drop dispensing bottles, and other similar forms of liquids and powders designed to be taken in measured small unit quantities.

In a preferred embodiment, the nutritional supplement composition according to the present invention contains 5 to 1000 mg, preferably 7.5 to 500 mg, especially 10 to 300 mg, of Zn complexed mammalian brain protein hydrolysate per ml in aqueous solution, or may be reconstituted to such an aqueous solution. The compositions according to the present invention are preferably provided in dry or in (aqueous) solution form. All references in the present disclosure relate to both, the dry form (eventually reconstituted to an aqueous form before (oral) administration) and the solution form. Reconstitution of a dry form of the Zn complexed mammalian brain protein hydrolysate is usually performed by adding water or an aqueous reconstitution solution to the dry product according to the instructions for use accompanying the nutritional supplement product.

Preferably, the nutritional supplement composition is in dose form wherein the dose contains 0.1 to 100 ml preferably 1 to 50 ml of Zn complexed mammalian brain protein hydrolysate, corresponding to 21.5 to 21,520 mg of Zn complexed mammalian brain protein hydrolysate in aqueous solution, or may be reconstituted to such an aqueous solution.

In a preferred embodiment, the nutritional supplement dose according to the present invention contains a total amount of Zn from 0.05 to 25 mg (per dose), preferably from 0.1 to 15 mg, especially from 0.5 to 10 mg.

In a preferred embodiment, the nutritional supplement composition according to the present invention has a pH in aqueous solution of 5.0 to 9.0. In the preferred "roughly neutral" embodiment, the pH of the composition is from 6.0 to 8.0, especially from 7.0 to 7.5, or is reconstitutable to an aqueous solution having a pH of 6.0 to 8.0, especially from 7.0 to 7.5. In the preferred "acidic" embodiment, the pH of the composition is from 3.0 to 6.0, preferably of 3.5 to 5.0, especially of 4.0 to 5.0. A specifically preferred nutritional supplement composition according to the present invention has a pH of about 4.7 (e.g. of 4.5 to 4.9) .

Preferably, in the nutritional supplement the zinc concentration in the Zn complexed mammalian brain protein hydrolysate has in aqueous solution a concentration of 0.05 to 50 mg/ml, preferably from 0.1 to 25 mg/ml, especially from 0.2 to 20 mg/ml, or wherein the Zn complexed mammalian brain protein hydrolysate is reconstitutable to an aqueous solution a concentration of 0.05 to 50 mg/ml, preferably from 0.1 to 25 mg/ml, especially from 0.2 to 20 mg/ml.

In a preferred dose form of the present nutritional supplement composition, the dose contains 5 to 1000 mg, preferably 7.5 to 500 mg, especially 10 to 300 mg, of Zn complexed mammalian brain protein hydrolysate.

In a preferred embodiment, the nutritional supplement composition according to the present invention contains a Zn complexed mammalian brain protein hydrolysate contains at least one peptide selected from the group NMVPFPR, ASAFQGIGSTHWVYDGVGNS, and DLHW. Such peptides are known for having high dietary value and improved neuroprotective activity (EP 0 452 299 A1, WO 2005/074970 A2, and WO 2007/128493 A1). This effect can even be increased by providing these peptides in Zn complexed form according to the present invention.

As already mentioned above, the Zn complexed mammalian brain protein hydrolysate is preferably dried, especially spray-dried, fluid bed dried or lyophilised. It may then be filled and finalised, especially to the respective dose form suitable for being marketed as a nutritional supplement. As a nutritional supplement, oral administrability and bioavailability by effective stomach passage are important elements. Accordingly, enteric coating represents a preferred embodiment of the products according to the present invention. Enteric coating is a well available technique for a person skilled in the art. Various substances, such as methyl acrylate-methacrylic acid copolymers, gelatine, collagen, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein, enteric coating aqueous solution (ethylcellulose, medium chain triglycerides (coconut), oleic acid, sodium alginate, stearic acid) (coated softgels), etc. are available to prevents dissolution or disintegration of the hydrolysate in the gastric environment. The Zn complexed mammalian brain protein hydrolysate according to the present invention is thereby protected from the acidity of the stomach and may be released after the stomach (e.g. in the upper tract of the intestine).

Therefore, the nutritional supplement in dry form according to the present invention is preferably provided as capsule, pastille, tablet, pill, or sachet of powder, especially as tablet or capsule with an enteric coating, for example a coating comprising methyl acrylate-methacrylic acid copolymers, gelatine, collagen, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein, enteric coating aqueous solution (ethylcellulose, medium chain triglycerides (coconut), oleic acid, sodium alginate, stearic acid) (coated softgels), or mixtures thereof.

Preferably, the nutritional supplement comprises, per dose, 10 mg to 5 g, preferably 50 mg to 1 g, especially 100 mg to 500 mg, of a carbohydrate as an additional substance, preferably a disaccharide, especially lactose, trehalose, or mixtures thereof.

According to a preferred embodiment of the present composition, especially if provided as a nutritional (dietary) supplement, the composition further comprises vitamins, such as vitamin A, different vitamins of the B group, vitamin C, vitamin D, E and/or K. Further it may comprise mineral substances and/or trace elements, such as calcium, magnesium, iron, copper, sodium, manganese, iodine, potassium, selenium, chromium, molybdenium, fluorine, chlorine, and/or phosphorous. Further ingredients may be caffeine and taurine, fatty acids, such as Q-fatty acids, alpha lipoic acid, phospholipids, phosphatidylserines, and/or plant extracts. It may further comprise flavouring substances, colorants, like titanium dioxide or ferric oxides, and/or preserving agents. Examples for such preserving are ethylparaben (p-Hydroxybenzoic acid ethyl ester), benzalkonium chloride, benzethonium chloride, benzoic acid, butylparaben (p-Hydroxybenzoic acid butyl ester), methylparaben (p-Hydroxybenzoic acid methyl ester), potassium sorbate, propionic acid, propylparaben (p-Hydroxybenzoic acid propyl ester), sodium benzoate, sodium propionate, sorbic acid, and/or mixtures thereof.

However, the present Zn complexed mammalian brain protein hydrolysate may also be used pharmaceutically, especially for the prevention and treatment of human diseases. Accordingly, the Zn complexed mammalian brain protein hydrolysate may also be finished to a pharmaceutically acceptable preparation, preferably by filling the Zn complexed mammalian brain protein hydrolysate into a pharmaceutically acceptable container.

According to another aspect, the present invention also refers to a pharmaceutical composition comprising the new Zn complexed mammalian brain protein hydrolysate provided by the present invention, preferably obtainable according to the method according to the present invention, and a pharmaceutically acceptable excipient.

A pharmaceutically acceptable excipient can be any excipient used in pharmaceutical formulations, such as a diluent, buffer, binder, stabiliser, preservative, etc..

The novel Zn complexed mammalian brain protein hydrolysate product according to the present invention can, of course, be applied in all the indications known or suggested for known mammalian brain protein hydrolysate products. Specifically preferred indications are (according to ICD11 nomenclature) cerebrovascular disease, preferably cerebral Ischemia (8B10, 8B11, 8B1Y/Z), especially intracranial haemorrhage (8B00-3), cerebrovascular disease with no acute cerebral symptom (8B21), certain specified cerebrovascular diseases (8B22), hypoxic-ischaemic encephalopathy (8B24), late effects of cerebrovascular disease (8B25), vascular syndromes of brain in cerebrovascular diseases (8B26), cerebrovascular diseases, unspecified (8B2Z); disorders with neurocognitive impairment as a major feature, preferably Alzheimer disease (8A20), progressive focal atrophies (8A21), Lewy body disease (8A22), frontotemporal lobar degeneration (8A23), other specified disorders with neurocognitive impairment as a major feature (8A2Y), disorders with neurocognitive impairment as a major feature, unspecified (8A2Z); headache disorders (8A80-85), preferably other specified headache disorders (8A8Y) and headache disorders, unspecified (8A8Z); motor neuron diseases or related disorders, preferably motor neuron disease (8B60), spinal muscular atrophy (8B61), post-polio progressive muscular atrophy (8B62), other specified motor neuron diseases or related disorders (8B6Y), and motor neuron diseases or related disorders, unspecified (8B6Z); injuries of the nervous system, especially injury of cranial nerves (NA04), intracranial injury (NA07), and crushing injury of head (NA08); hypoxic ischaemic encephalopathy of newborn (8B24KB04), spinal cord disorders excluding trauma, disorders of nerve root, plexus or peripheral nerves, diseases of neuromuscular junction or muscle, disorders of nerve root, plexus or peripheral nerves, cerebral palsy, disorders of autonomic nervous system, and postprocedural disorders of the nervous system.

Preferred further medical indications are age associated memory impairment, age associated cognitive decline, mental agility support, compromised age associated microvascular patency, protection of cell constituents (DNA, Proteins and Lipids) from oxidative damage, normalization of immune system and vascular function, improvement of neuron vitality and recovery, support of nerve growth and connection, enhancing cognitive function and activating bioelectrical activity, preferably in healthy subjects, especially in healthy elderly subjects (age: 51+), protecting against age and disease associated lesions in cortical neurons (Alvarez et al., Meth. Find. Exp. Clin. Pharmacol. 27 (20005), 483-487; Vole, Neurol. Psychiatrie (2005), 1-7; Crook et al., Int. Clin.Psychopharm. 20 (2005), 97-100; Windisch et al., J. Neural Transm. 112 (2005), 1331-1343).

The compositions according to the present invention can also be used in a non-therapeutic environment. In this use, the compositions according to the present invention are administered to healthy subjects, i.e. persons not having a disease or disorder. Preferably, the compositions according to the present invention are administered for enhancing cognitive function and activating bioelectrical activity in healthy subjects, especially in healthy elderly subjects (age: 51+). Preferably the (nutritional) compositions according to the present invention are useful to prevent, ameliorate, and/or counteract deficiencies related with the aging process preferably in mammals, especially in humans, most preferably in elderly humans

Preferred concentrations to be used according to the present invention contain 50 to 1000 mg, preferably 100 to 500 mg, especially 100 to 250 mg, of Zn complexed mammalian brain protein hydrolysate according to the present invention per ml in aqueous solution.

A preferred method for producing mammalian brain protein hydrolysate used as a basis for the complexing method according to the present invention is disclosed in EP 0 452 299 A1 and is obtained from enzymatic hydrolysis of swine brain protein fraction.

The dose applied may preferably be in the range of 10 to 10 000 mg (0.5 to 50 ml Zn complexed mammalian brain protein hydrolysate). Preferably, treatment is performed with a dose in the range of 0.1 to 100 ml, preferably 1 to 50 ml, of Zn complexed mammalian brain protein hydrolysate, (a preferred Zn complexed mammalian brain protein hydrolysate has a concentration between 50 to 1000 mg/ml, preferably 75 to 500 mg/ml, especially 100 to 300 mg/ml). If applied intramuscularly, the dose is usually lower than intravenously (e.g., preferably 0.5 to 5 ml and preferably 0.5 to 10 ml, intravenously). Preferably, the treatment is performed by an intramuscularly administered dose of 0.1 to 10 ml, preferably 0.5 to 5 ml, corresponding to 21.5 mg to 2152 mg Zn complexed mammalian brain protein hydrolysate. Zn complexed mammalian brain protein hydrolysate may also be continuously infused (usually at volumes above 10 ml) and the Zn complexed mammalian brain protein hydrolysate may therefore e.g. be diluted with 0.9% sodium chloride solution (9 mg NaCl/ml), Ringer's solution (Na⁺ 153.98 mmol/l, Ca²⁺ 2,74 mmol/l, K⁺ 4,02 mmol/l, Cl⁻ 163,48 mmol/l), 5 % glucose. Infusion durations of typically 5 min to 4 h, preferably 10 min to 2 h, especially 15 to 60 min may be performed (preferably each day) e.g. for 1 to 100 d, preferably from 5 to 50 d, preferably from 10 to 30 d (usually, the administration is performed only on working days, not on weekends, so that usually a treatment cycle is applied in 3-5 x 5 consecutive days of administration) . According to a preferred embodiment, the treatment is performed with an intravenously administered dose of 0.1 to 100 ml, preferably 1 to 50 ml, corresponding to 215.2 to 21,520 mg Zn complexed mammalian brain protein hydrolysate. In a preferred embodiment, the pharmaceutical preparation according to the present invention contains a total amount of Zn from 0.1 to 25 mg (per dose), preferably from 0.5 to 10 mg, especially from 0.5 to 5 mg.

Treatment cycles can be repeated after a treatment-free period from 1 to 6, from 1 to 3, preferably 2 to 3, months.

The present invention is further described in the following examples and the figures, yet without being limited thereto.

Fig. 1 shows size exclusion chromatograms of mammalian brain protein hydrolysate and water-soluble protein hydrolysate - Zn complexes formed by addition of 2 or 4 mg zinc gluconate (ZnGlu)/mg N₂ in the mammalian brain protein hydrolysate.

Fig. 2 shows the enlarged part of the SE-chromatograms showing, upon complexation, a shift in the peak positions to shorter retention times, i.e. higher molecular weights, resulting from the binding of zinc ions to the peptide molecules. In addition, when using 4 mg rather than 2 mg ZnGlu/mg N₂ in the mammalian brain protein hydrolysate, the peak at RT ~ 52.5 min loses in intensity and an additional small peak appears at RT ~ 48 min. Amino acids elute in the peak at RT ~ 58 min. The minor shift to lower RT shows the formation of amino acid-zinc complexes.

Fig. 3 shows a dose response of NPEP-Z on BBB permeability.

Fig. 4 shows Western Blot data of NPEP-Z on endothelial proteins.

Fig. 5 shows Western Blot data of NPEP-Z on endothelial proteins under tPA condition.

Fig. 6 shows Western Blot data of NPEP-Z on endothelial proteins under fibrin condition.

Fig. 7 shows the percentage of p75NTR expression on surfaces of differently treated PC12 Cells relative to medium treated Cells. PC12 Cells were cultivated for 24 hours with 100 µl/ml N-PEP-Z or Zn only dilutions as indicated and analysed for the expression of p75NTR using flow cytometry.

### Examples

### Preparation of mammalian brain protein hydrolysate - zinc complexes

### - Lab experiment

An aqueous solution of zinc gluconate (10 ml, 350 - 750 mg) was added with stirring to a mammalian brain protein hydrolysate solution (20 ml, 7-15mg/ml nitrogen (N₂), pH 2-7). The pH changed, indicative of complex formation. After adjustment to neutral, the suspension was used for analysis.

### - Pilot experiment

Mammalian brain protein hydrolysate solution (40 l) was adjusted to pH 3 using 6N HCl. Zinc gluconate (858 g) was added with stirring. The obtained clear solution was sterile filtered. 40 ml of the solution was adjusted to pH 7.2 and then centrifuged before analysis.

### Analytical characterization

To determine the percentage of zinc forming water soluble complexes with peptide and amino acids, the supernatant was subjected to size exclusion chromatography. The collected fractions were analyzed for zinc using microwave digestion/ICP-MS.

To determine the percentage of peptides and free amino acids in the water-soluble fraction and in the precipitate, total nitrogen content and free amino acid concentration were determined both in the supernatant and in the mammalian brain protein hydrolizate.

For size exclusion chromatography, a Superdex Peptide 10/300 column was used. 50 µL of the filtered supernatant was eluted with 100 mM ammonium acetate in 30% acetonitrile at a flow rate of 0.4 ml/min. UV detection was at 214 nm. The eluate was collected in three fractions and analyzed for zinc. Ribonuclease A, somatostatin and serine were used for column calibration.

For nitrogen analysis, the Flash Dynamic Combustion method was used. The determination of the concentration of the individual free amino acids was based on pre-column derivatization RP-HPLC.

Nitrogen content of peptides, calculated by subtracting the nitrogen content of all amino acids from the total nitrogen content, was used as a surrogate for peptide content.

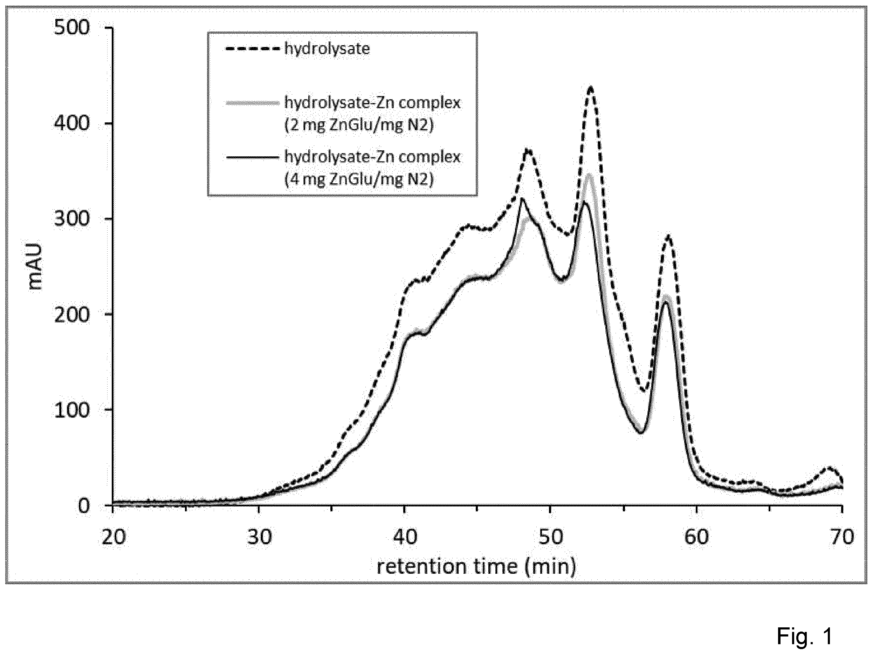

Fig. 1 shows the size exclusion chromatograms of mammalian brain protein hydrolysate and the water-soluble fraction of protein hydrolysate - Zn complexes formed by addition of 2 or 4 mg Zn gluconate (ZnGlu)/mg nitrogen (N2) in the mammalian brain protein hydrolysate.

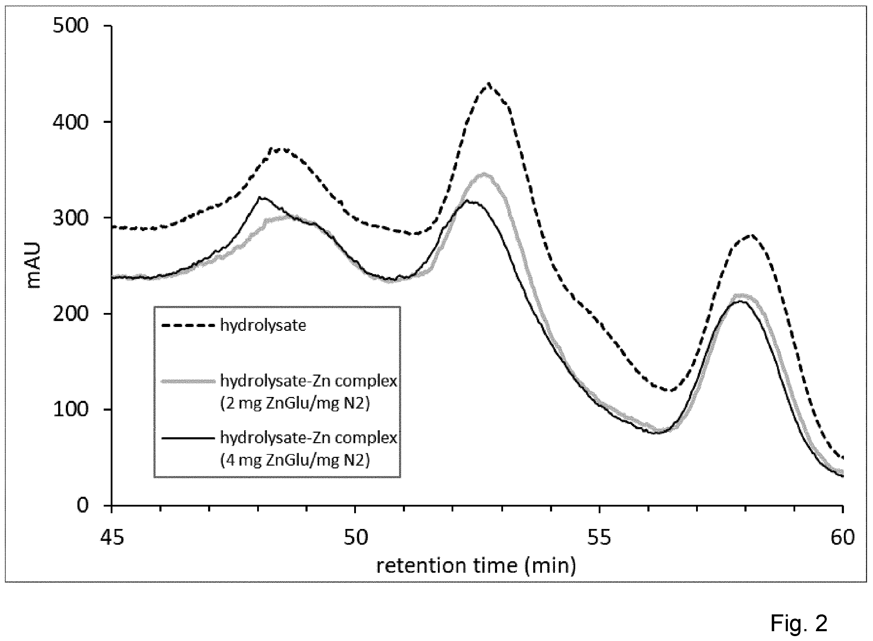

Fig. 2 shows the enlarged part of the SE-chromatograms showing, upon complexation, a tiny shift in the peak positions to shorter retention times, i.e. higher molecular weights, resulting from the binding of zinc ions to the peptide molecules. In addition, when using 4 mg rather than 2 mg Zn gluconate (ZnGlu)/mg N₂ in the protein hydrolysate, the peak at RT ~ 52.5 min loses in intensity and an additional small peak appears at RT ~ 48 min. Amino acids elute in the peak at RT ~ 58 min. The minor shift to lower RT shows the formation of amino acid-zinc complexes.

**Tab. 1. Percentage of zinc found in the individual SEC fractions of water-soluble mammalian brain protein hydrolysate - zinc-complexes when using 2, 4 (both lab experiments) or 2.5 (pilot experiment) mg ZnGlu/mg N₂ in the protein hydrolysate.**

| | | | lab experiment | | pilot experiment |
|---|---|---|---|---|---|
| # no. composition | | RT (min) | % Zn | % Zn | % Zn |
| | | | (2 mg ZnGlu/mg N₂) | (4 mg ZnGlu/mg N₂) | (2.5 mg ZnGlu/mg N₂) |
| 1 | peptides | 30 - 50 | 15.2 | 27.8 | 27.3 |
| 2 | peptides | 50 - 55 | 24.4 | 26.8 | 27.7 |
| 3 | amino acids | 55 - 60 | 4.4 | 3.1 | 3.0 |
| | soluble Zn | | 44.0 | 57.7 | 58.0 |

**Tab. 2. Percentage of amino acids and peptides found in the individual SEC fractions of water-soluble mammalian brain protein hydrolysate -zinc complexes when using 2, 4 (both lab experiments) or 2.5 (pilot experiment) mg ZnGlu/mg N₂ in the protein hydrolysate.**

| | lab experiment | | pilot experiment |
|---|---|---|---|
| | (2 mg ZnGlu/mg N₂) | (4 mg ZnGlu/mg N₂) | (2.5 mg ZnGlu/mg N₂) |
| a amino acids | 92.9 | 85.4 | 87.3 |
| a peptides | 89.1 | 85.5 | 81.2 |

### Effects of peptide-zinc complex on human neuroendothelial cells in vitro.

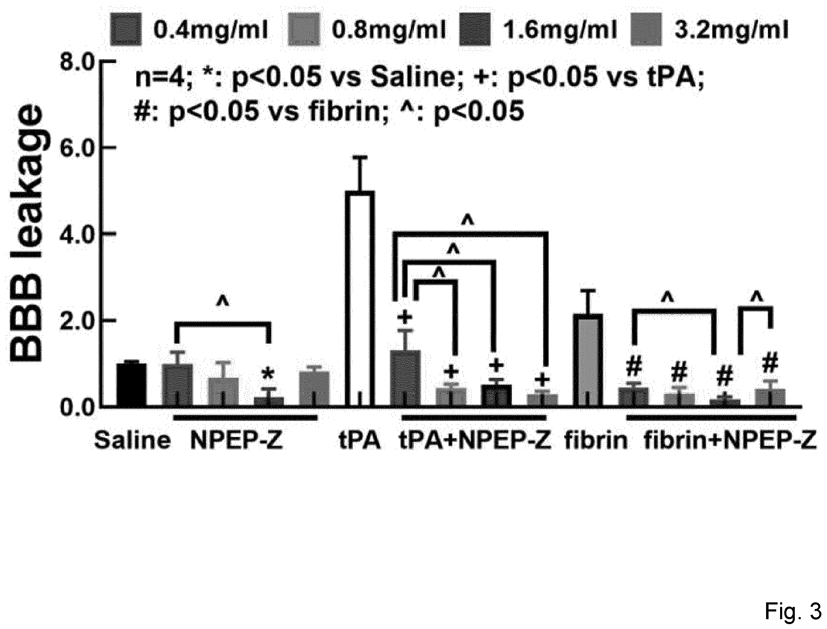

To investigate a dose response of N-PEP-Z on transendothelial permeability, human cerebral endothelial cells were seeded in the insert of a transwell and FITC-dextran was added into the inserted well (Li et al., Stroke 50 (2019), 2547-2554**).** The cerebral endothelial cells were treated with tissue plasminogen activator (tPA) (10µl/ml), or fibrin (1.5pl/ml) in the presence or absence of N-PEP-Z or controls. FITC-dextran signal in the main well were quantified as an index of transendothelial permeability. Doses of N-PEP-Z were adapted from preclinical study with N-PEP-12 (Windisch et al., J. Neural Transm. 112 (2004), 1331-1343). FITC signals were measured in the individual main well after transwell removal. The FITC signals in the control group were then normalized to 1 and any changes in the individual intervention groups were calculated and compared to the control group. Data in Figs. 3 to 6 are presented as fold changes of BBB permeability vs the control (y-axis) .

### Effect of N-PEP-Z on inflammatory cytokines and tight junction proteins

Cerebral endothelial cells were treated with tPA and fibrin in the presence or absence of N-PEP-Z for 24h. After treatment, total proteins were extracted from the treated endothelial cells and Western blot analysis was performed to quantify changes of proteins. Proteins to induce thrombosis, inflammation and vascular injury were examined (including TNFα, intercellular adhesion molecule 1 (ICAM1), high mobility group protein B1 (HMGB1) and NFκB) as well as proteins mediating functional integrity of the blood brain barrier such as, Such as Zonula Occludens 1, (ZO1), Occludin, and Claudin 5.

### Results

It was found that all tested doses of N-PEP-Z significantly reduced endothelial permeability induced by tPA or Fibrin (Fig. 3) whereas in unstressed condition 1.6 mg/ml of N-PEP-Z significantly reinforced endothel integrity and reduced inflammation.

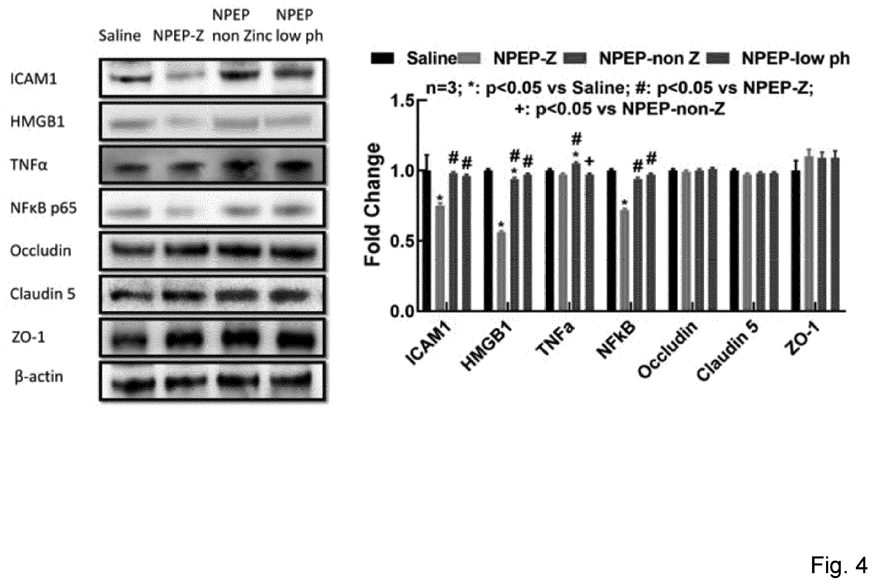

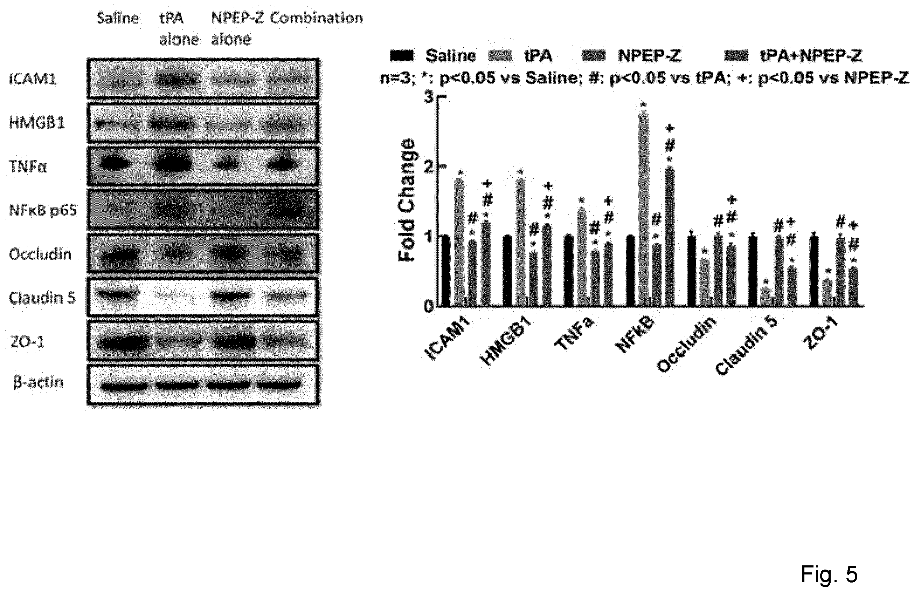

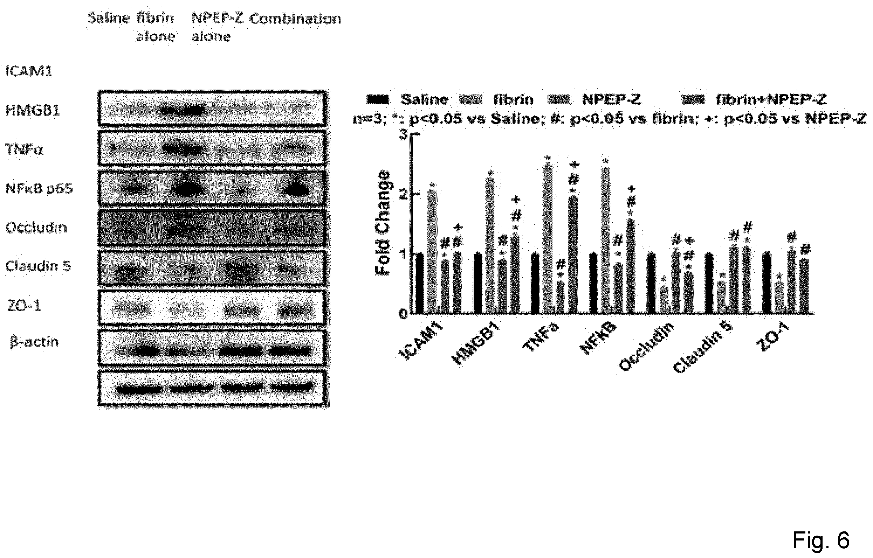

In addition, it was found that compared with saline, NPEP-non-Z (no Zinc) and NPEP-low pH (no Peptide-Zinc complex formation) did not significantly alter pro-inflammatory proteins without the tPA or Fibrin stress. However, NPEP-Zinc significantly reduced pro-inflammatory proteins of ICAM1, HMGB1, and active NFκB compared with saline, NPEP-non-Zinc and NPEP-low pH whereas markers of Blood Brain Barrier integrity remained unaffected (Fig. 4). tPA (Fig. 5) or fibrin (Fig. 6) significantly increased ICAM1, HMGB1, TNFα, and active NFκB and reduced tight junction proteins, ZO1, Occludin, and Claudin 5. NPEP-Z (lowest concentration tested in Figure 3) significantly reversed tPA- (Fig. 5) or fibrin-altered (Fig. 6) proteins.

The observed positive impact of N-PEP zinc according to the present invention on the basal state of inflammation (no tPA or fibrin stress) shows the use of this composition in non-pathological conditions having a strong contribution of inflammatory components. Such changes occur during normal aging and are associated with cognitive decline in the elderly (Simen et al. Ther. Adv. Chronic. Dis. (2011), 175-195) but also during stress induced damage of the hippocampal formation (Gulyaeva et al., Biochemistry, (2019), 1306-1328).

### Effects of N-PEP-Z on surface expression of p75NTR in PC12 Cells

The neurotrophin receptor p75NTR is a member of the TNF receptor superfamily and involved in the regulation of survival of neurons. The receptor is known to be upregulated under pathologic conditions mediating apoptosis (for review see Dechant G. et al., Nature Neuroscience 2002). Many studies show the upregulation of p75NTR during Stroke or Traumatic Brain Injury (Grade S. et al., PlosOne 2013; Irmady K. et al., J. of Neurosci. 2014; Shi J. et al., Stem Cell 2013), as well as in Alzheimer's disease (Y Hu et al., Cell and Disease 2013; S. Ito et al., Neurochemistry International 2016; B. Charkravathy et al., Journal of Alzheimer's disease 2009) and several other neurodegenerative diseases (for review see Meeker R. et al., J Neuroimmune Pharmacol. 2014).

### Method

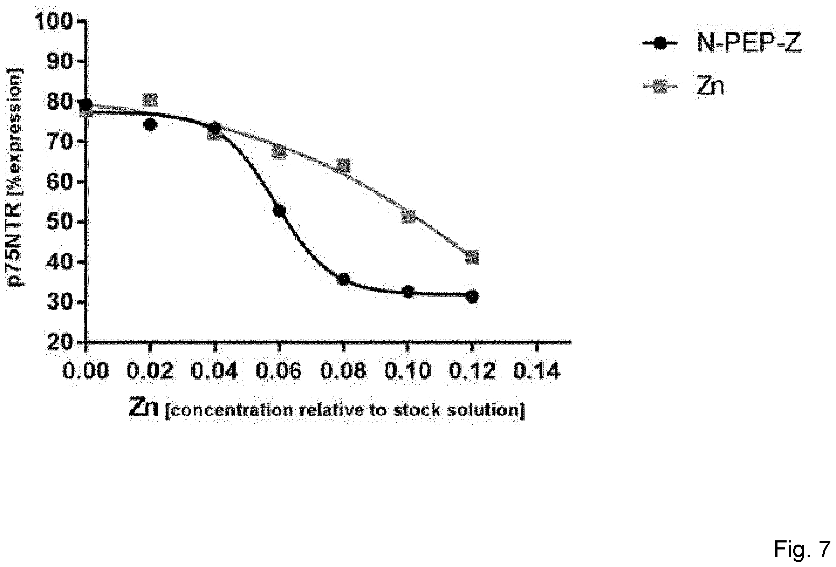

To investigate a putative effect of N-PEP-Z on p75NTR-receptor expression, PC12 Cells were seeded into 6-well plates and treated with N-PEP-Z comprising Zn-concentrations from 0 to 0.12 relative to N-PEP-Z stock solution (31,84 mM Zn). The dilutions were prepared as described in Table 1 (a Zn-gluconate solution was diluted the same way in PBS, serving as "Zn only control"). The PC12 Cells were treated with 100 µl/ml of the respective dilutions for 24h. After treatment, PC12 cells were collected for surface staining with Alexa-647 conjugated anti-p75NTR antibody. Alexa-647 signals were measured using flow cytometry and normalized to medium treated PC12 Cells (% expression). Data in Figure 7 is presented as % expression of p75NTR receptor (y-axis).

**Tab. 3: Used dilutions for the N-PEP-Z treatment**

| | 1x N-PEP [µL] | 1x N-PEP-Z [µL] |
|---|---|---|
| N-PEP 0.12x Zn | 880 | 120 |
| N-PEP 0.10x Zn | 900 | 100 |
| N-PEP 0.08x Zn | 920 | 80 |
| N-PEP 0.06x Zn | 940 | 60 |
| N-PEP 0.04× Zn | 960 | 40 |
| N-PEP 0.02x Zn | 980 | 20 |
| N-PEP 0.00x Zn | 1000 | 0 |

### Results

Dose response curve of N-PEP-Z showed a significant reduction of p75NTR surface expression compared to Zn only treatment. The inflection point of the dose response curve was determined at 0.06x Zn (Figure 7).

In this study the downregulation of the p75NTR receptor by N-PEP-Z complexes on PC12 cell surfaces was investigated. These data show the protective activity of N-PEP-Z complexes according to the present invention during neurodegenerative disease progression by regulating elevated p75NTR levels.

## Claims

1. Method for producing a mammalian brain protein hydrolysate composition, comprising the following steps:
- providing a mammalian brain protein hydrolysate,
- adding Zn ions to the mammalian brain protein hydrolysate to form a complex comprising the Zn ions and the mammalian brain protein hydrolysate,
- obtaining the complex comprising the Zn ions and the mammalian brain protein hydrolysate in a mammalian brain protein hydrolysate composition.

2. Method according to claim 1, wherein the mammalian brain protein hydrolysate is a hydrolysate of porcine or bovine brain tissue, preferably an intermediate of the brain protein hydrolysate manufacturing process, especially a peptide-enriched intermediate product.

3. Method according to claim 1 or 2, wherein the Zn ions are added as such as Zn gluconate, Zn sulfate or Zn acetate, preferably as Zn gluconate, and preferably in amount of 0.1 to 500 mg/ml, preferably from 0.5 to 100 mg/ml, especially from 1 to 70 mg/ml.

4. Method according to any one of claims 1 to 3, wherein the mammalian brain protein hydrolysate is an enzymatically hydrolysated mammalian brain protein hydrolysate, preferably hydrolysated by pancreatin, trypsin, pepsin, chymotrypsin, papain, carboxypeptidase, or mixtures thereof.

5. Method according to any one of claims 1 to 4, wherein the pH of the mammalian brain protein hydrolysate composition is adjusted upon formation of the complex comprising the Zn ions and the mammalian brain protein hydrolysate to pH of 3.0 to 9.0, preferably
(a) to a pH of 5.0 to 9.0, more preferred to a pH of 6.0 to 8.0, especially to a pH of 7.0 to 7.5; or
(b) to a pH of 3.0 to 6.0, more preferred to a pH of 3.0 to 5.0, especially to a pH of 4.0 to 5.0 and/or to a pH of about 4.7, namely of 4.5 to 4.9.

6. Method according to any one of claims 1 to 5, wherein the Zn ions are added to the mammalian brain protein hydrolysate in a relative amount of 0.01 to 50 mg Zn per mg mammalian brain protein hydrolysate (as mg N₂), preferably of 0.05 mg to 10 mg Zn per mg mammalian brain protein hydrolysate (as mg N₂), especially 0.1 mg to 4 mg Zn per mg mammalian brain protein hydrolysate (as mg N₂).

7. Method according to any one of claims 1 to 6, wherein the Zn complexed mammalian brain protein hydrolysate is adjusted to a concentration of 0.05 to 50 mg/ml, preferably from 0.1 to 25 mg/ml, especially from 0.2 to 20 mg/ml.

8. Method according to any one of claims 1 to 7, wherein the Zn complexed mammalian brain protein hydrolysate is finished to a final preparation, preferably to a nutritional supplement or to a pharmaceutically acceptable preparation, especially by filling the Zn complexed protein hydrolysate into a nutritionally acceptable dosage form or into a pharmaceutically acceptable container.

9. Method according to any one of claims 1 to 8, wherein the Zn complexed mammalian brain protein hydrolysate is dried, preferably spray-dried, fluid bed dried or lyophilised.

10. Composition comprising a Zn complexed mammalian brain protein hydrolysate, preferably obtainable according to any one of claims 1 to 9.

11. Nutritional supplement comprising a Zn complexed mammalian brain protein hydrolysate, preferably obtainable according to any one of claims 1 to 9.

12. Nutritional supplement according to claim 11 in dose form, preferably as capsules, pastilles, tablets, pills and other similar forms, sachets of powder, ampoules of liquids, drop dispensing bottles, and other similar forms of liquids and powders designed to be taken in measured small unit quantities.

13. Nutritional supplement according to claim 11 or 12, wherein the composition contains 5 to 1000 mg, preferably 7.5 to 500 mg, especially 10 to 300 mg, of Zn complexed mammalian brain protein hydrolysate per ml in aqueous solution, or may be reconstituted to such an aqueous solution.

14. Pharmaceutical composition comprising a Zn complexed mammalian brain protein hydrolysate, preferably obtainable according to any one of claims 1 to 9, and a pharmaceutically acceptable excipient.

15. Pharmaceutical composition according to claims 14, for use in a therapeutic treatment, wherein the composition contains Zn complexed mammalian brain protein hydrolysate.

## Patentansprüche

1. Verfahren zur Herstellung einer Säugetierhirnproteinhydrolysat-Zusammensetzung, aufweisend die folgenden Schritte:
- Bereitstellen eines Säugetierhirnproteinhydrolysats,
- Zugeben von Zn-Ionen zu dem Säugetierhirnproteinhydrolysat, um einen Komplex zu bilden, der die Zn-Ionen und das Säugetierhirnproteinhydrolysat aufweist,
- Erhalten des Komplexes, der die Zn-Ionen und das Säugetierhirnproteinhydrolysat aufweist, in einer Säugetierhirnproteinhydrolysat-Zusammensetzung.

2. Verfahren nach Anspruch 1, wobei das Säugetierhirnproteinhydrolysat ein Hydrolysat von Schweine- oder Rinderhirngewebe ist, vorzugsweise ein Zwischenprodukt des Hirnproteinhydrolysat-Herstellungsverfahrens, insbesondere ein Peptid-angereichertes Zwischenprodukt.

3. Verfahren nach Anspruch 1 oder 2, wobei die Zn-Ionen an sich als Zn-Gluconat, Zn-Sulfat oder Zn-Acetat, vorzugsweise als Zn-Gluconat, und vorzugsweise in einer Menge von 0,1 bis 500 mg/ml, vorzugsweise von 0,5 bis 100 mg/ml, insbesondere von 1 bis 70 mg/ml, zugegeben werden.

4. Verfahren nach einem der Ansprüche 1 bis 3, wobei das Säugetierhirnproteinhydrolysat ein enzymatisch hydrolysiertes Säugetierhirnproteinhydrolysat ist, das vorzugsweise durch Pankreatin, Trypsin, Pepsin, Chymotrypsin, Papain, Carboxypeptidase oder Mischungen davon hydrolysiert wurde.

5. Verfahren nach einem der Ansprüche 1 bis 4, wobei der pH-Wert der Säugetierhirnproteinhydrolysat-Zusammensetzung bei der Bildung des Komplexes, der die Zn-Ionen und das Säugetierhirnproteinhydrolysat aufweist, auf einen pH-Wert von 3,0 bis 9,0, vorzugsweise
(a) auf einen pH-Wert von 5,0 bis 9,0, bevorzugter auf einen pH-Wert von 6,0 bis 8,0, insbesondere auf einen pH-Wert von 7,0 bis 7,5, oder
(b) auf einen pH-Wert von 3,0 bis 6,0, bevorzugter auf einen pH-Wert von 3,0 bis 5,0, insbesondere auf einen pH-Wert von 4,0 bis 5,0 und/oder auf einen pH-Wert von etwa 4,7, nämlich von 4,5 bis 4,9, eingestellt ist.

6. Verfahren nach einem der Ansprüche 1 bis 5, wobei die Zn-Ionen zu dem Säugetierhirnproteinhydrolysat in einer relativen Menge von 0,01 bis 50 mg Zn pro mg Säugetierhirnproteinhydrolysat (als mg N₂), vorzugsweise von 0,05 mg bis 10 mg Zn pro mg Säugetierhirnproteinhydrolysat (als mg N₂), insbesondere 0,1 mg bis 4 mg Zn pro mg Säugetierhirnproteinhydrolysat (als mg N₂), zugegeben werden.

7. Verfahren nach einem der Ansprüche 1 bis 6, wobei das Znkomplexierte Säugetierhirnproteinhydrolysat auf eine Konzentration von 0,05 bis 50 mg/ml, vorzugsweise 0,1 bis 25 mg/ml, insbesondere 0,2 bis 20 mg/ml, eingestellt ist.

8. Verfahren nach einem der Ansprüche 1 bis 7, wobei das Znkomplexierte Säugetierhirnproteinhydrolysat zu einem Endpräparat, vorzugsweise zu einem Nahrungsergänzungsmittel oder zu einem pharmazeutisch annehmbaren Präparat, fertiggestellt wird, insbesondere durch Abfüllen des Zn-komplexierten Proteinhydrolysats in eine ernährungsphysiologisch annehmbare Darreichungsform oder in einen pharmazeutisch annehmbaren Behälter.

9. Verfahren nach einem der Ansprüche 1 bis 8, wobei das Znkomplexierte Säugetierhirnproteinhydrolysat getrocknet, vorzugsweise sprühgetrocknet, in einer Wirbelschicht getrocknet oder lyophilisiert wird.

10. Zusammensetzung, aufweisend ein Zn-komplexiertes Säugetierhirnproteinhydrolysat, vorzugsweise erhältlich nach einem der Ansprüche 1 bis 9.

11. Nahrungsergänzungsmittel aufweisend ein Zn-komplexiertes Säugetierhirnproteinhydrolysat, vorzugsweise erhältlich nach einem der Ansprüche 1 bis 9.

12. Nahrungsergänzungsmittel nach Anspruch 11 in Dosierungsform, vorzugsweise als Kapseln, Pastillen, Tabletten, Pillen und weitere ähnliche Formen, Beutel mit Pulver, Ampullen mit Flüssigkeiten, Flaschen mit Tropfeinsätzen und weitere ähnliche Formen von Flüssigkeiten und Pulvern zum Einnehmen in abgemessenen kleinen Mengen.

13. Nahrungsergänzungsmittel nach Anspruch 11 oder 12, wobei die Zusammensetzung 5 bis 1 000 mg, vorzugsweise 7,5 bis 500 mg, insbesondere 10 bis 300 mg Zn-komplexiertes Säugetierhirnproteinhydrolysat pro ml in wässriger Lösung enthält oder zu einer solchen wässrigen Lösung rekonstituiert werden kann.

14. Pharmazeutische Zusammensetzung, aufweisend ein Zn-komplexiertes Säugetierhirnproteinhydrolysat, vorzugsweise erhältlich nach einem der Ansprüche 1 bis 9, und einen pharmazeutisch annehmbaren Hilfsstoff.

15. Pharmazeutische Zusammensetzung nach Anspruch 14 zur Anwendung bei einer therapeutischen Behandlung, wobei die Zusammensetzung Zn-komplexiertes Säugetierhirnproteinhydrolysat enthält.

## Revendications

1. Procédé pour produire une composition d'hydrolysat de protéines cérébrales de mammifère, comprenant les étapes suivantes:
- fourniture d'un hydrolysat de protéines cérébrales de mammifère,
- addition d'ions Zn à l'hydrolysat de protéines cérébrales de mammifère pour former un complexe comprenant les ions Zn et l'hydrolysat de protéines cérébrales de mammifère,
- obtention du complexe comprenant les ions Zn et l'hydrolysat de protéines cérébrales de mammifère dans une composition d'hydrolysat de protéines cérébrales de mammifère.

2. Procédé selon la revendication 1, dans lequel l'hydrolysat de protéines cérébrales de mammifère est un hydrolysat de tissu cérébral porcin ou bovin, de préférence un intermédiaire du procédé de fabrication d'hydrolysat de protéines cérébrales, en particulier un produit intermédiaire enrichi en peptides.

3. Procédé selon la revendication 1 ou 2, dans lequel les ions Zn sont ajoutés sous forme de gluconate de Zn, de sulfate de Zn ou d'acétate de Zn, de préférence sous forme de gluconate de Zn, et de préférence en une quantité de 0,1 à 500 mg/ml, de préférence de 0,5 à 100 mg/ml, en particulier de 1 à 70 mg/ml.

4. Procédé selon l'une quelconque des revendications 1 à 3, dans lequel l'hydrolysat de protéines cérébrales de mammifère est un hydrolysat de protéines cérébrales de mammifère hydrolysé par voie enzymatique, de préférence hydrolysé par la pancréatine, la trypsine, la pepsine, la chymotrypsine, la papaïne, la carboxypeptidase ou des mélanges de celles-ci.

5. Procédé selon l'une quelconque des revendications 1 à 4, dans lequel le pH de la composition d'hydrolysat de protéines cérébrales de mammifère est ajusté lors de la formation du complexe comprenant les ions Zn et l'hydrolysat de protéines cérébrales de mammifère à un pH de 3,0 à 9,0, de préférence
(a) à un pH de 5,0 à 9,0, de préférence encore à un pH de 6,0 à 8,0, en particulier à un pH de 7,0 à 7,5; ou
(b) à un pH de 3,0 à 6,0, de préférence encore à un pH de 3,0 à 5,0, en particulier à un pH de 4,0 à 5,0 et/ou à un pH d'environ 4,7, à savoir de 4,5 à 4,9.

6. Procédé selon l'une quelconque des revendications 1 à 5, dans lequel les ions Zn sont ajoutés à l'hydrolysat de protéines cérébrales de mammifère en une quantité relative de 0,01 à 50 mg de Zn par mg d'hydrolysat de protéines cérébrales de mammifère (sous forme de mg de N₂), de préférence de 0,05 mg à 10 mg de Zn par mg d'hydrolysat de protéines cérébrales de mammifère (sous forme de mg de N₂), en particulier de 0,1 mg à 4 mg de Zn par mg d'hydrolysat de protéines cérébrales de mammifère (sous forme de mg de N₂).

7. Procédé selon l'une quelconque des revendications 1 à 6, dans lequel l'hydrolysat de protéines cérébrales de mammifère complexé à Zn est ajusté à une concentration de 0,05 à 50 mg/ml, de préférence de 0,1 à 25 mg/ml, en particulier de 0,2 à 20 mg/ml.

8. Procédé selon l'une quelconque des revendications 1 à 7, dans lequel l'hydrolysat de protéines cérébrales de mammifère complexé à Zn est fini en une préparation finale, de préférence en un complément nutritionnel ou en une préparation acceptable du point de vue pharmaceutique, en particulier par introduction de l'hydrolysat de protéines complexées à Zn dans une forme posologique acceptable du point de vue nutritionnel ou dans un récipient acceptable du point de vue pharmaceutique.

9. Procédé selon l'une quelconque des revendications 1 à 8, dans lequel l'hydrolysat de protéines cérébrales de mammifère complexé à Zn est séché, de préférence séché par pulvérisation, séchés sur lit fluidisé ou lyophilisé.

10. Composition comprenant un hydrolysat de protéines cérébrales de mammifère complexé à Zn, de préférence susceptible d'être obtenu selon l'une quelconque des revendications 1 à 9.

11. Complément nutritionnel comprenant un hydrolysat de protéines cérébrales de mammifère complexé à Zn, de préférence susceptible d'être obtenu selon l'une quelconque des revendications 1 à 9.

12. Complément nutritionnel selon la revendication 11 sous forme posologique, de préférence sous forme de gélules, de pastilles, de comprimés, de pilules et d'autres formes similaires, de sachets de poudre, d'ampoules de liquides, de flacons compte-gouttes, et d'autres formes similaires de liquides et de poudres conçues pour être prises en petites quantités unitaires mesurées.

13. Complément nutritionnel selon la revendication 11 ou 12, dans lequel la composition contient 5 à 1000 mg, de préférence 7,5 à 500 mg, en particulier 10 à 300 mg, d'hydrolysat de protéines cérébrales de mammifère complexé à Zn par ml en solution aqueuse, ou peut être reconstitué en une telle solution aqueuse.

14. Composition pharmaceutique comprenant un hydrolysat de protéines cérébrales de mammifère complexé à Zn, de préférence susceptible d'être obtenu selon l'une quelconque des revendications 1 à 9, et un excipient pharmaceutiquement acceptable.

15. Composition pharmaceutique selon la revendication 14, pour utilisation dans un traitement thérapeutique, dans laquelle la composition contient un hydrolysat de protéines cérébrales de mammifère complexé à Zn.
